# FASCICULE DE BREVET EUROPEEN

(11) **EP 1 978 894 B1**
(45) Date de publication et mention de la délivrance du brevet: **03.11.2010**
(21) Numéro de dépôt: 06841822.7
(22) Date de dépôt: 29.11.2006
(51) Int. Cl.: A61F 2/08

(54) **IMPLANT CHIRURGICAL A APPUI EXTRA CORTICAL POUR TRANSPLANT LIGAMENTAIRE**
CHIRURGISCHES IMPLANTAT MIT EXTRAKORTIKALER UNTERSTÜTZUNG FÜR EIN LIGAMENTUM-TRANSPLANTAT
SURGICAL IMPLANT WITH EXTRACORTICAL SUPPORT FOR A LIGAMENT TRANSPLANT

(30) Priorité: 29.11.2005 FR 0512075
(43) Date de publication de la demande: 15.10.2008
(73) Titulaire: Imbert, Pierre, 83600 Fréjus (FR)
(72) Inventeur: Imbert, Pierre, 83600 Fréjus (FR)
(74) Mandataire: Bonneau, Gérard
(86) Numéro de dépôt international: PCT/FR2006/002611
(87) Numéro de publication internationale: WO 2007/063213

(56) Documents cités:
- EP-A- 0 933 064
- EP-B- 1 146 834
- FR-A- 2 774 582
- US-A- 6 056 752

## Description

### Domaine technique

La présente invention concerne le domaine des interventions chirurgicales destinées à remplacer un ligament dans une articulation et concerne en particulier un implant chirurgical à appui extra cortical pour transplant ligamentaire.

### Etat de la technique

Les articulations entre deux os comportent généralement un ou plusieurs ligaments reliant les deux os de part et d'autre de l'articulation. Les efforts importants auxquels sont soumis ces ligaments peuvent malheureusement provoquer leur rupture. Ceci est le cas de ligaments croisés du genou très sollicités dans les sports intensifs comme le football.

Lorsqu'un ligament tel qu'un ligament croisé antérieur du genou s'est rompu, il est possible de le remplacer en pratiquant une intervention chirurgicale appelée ligamentoplastie qui consiste à fixer un nouveau ligament appelé transplant aux extrémités d'un tunnel s'étendant de part et d'autre de l'articulation. Le tunnel d'une longueur d'environ 10cm et d'un diamètre compris entre 7mm et 12mm est percé à partir d'un des deux os et s'étend dans l'autre os sur une profondeur suffisante. Le ligament de remplacement peut être un ligament artificiel ou une partie de ligament prélevée sur un ligament d'une autre partie du corps ou sur un cadavre.

La fixation du transplant aux extrémités du tunnel doit être solide du fait qu'elle constitue le point faible de la reconstruction du ligament dans les premières semaines post opératoires. Ensuite, la repousse osseuse autour du transplant maintiendra celui-ci de façon solide et définitive (phase d'ostéo-intégration).

La résistance de la fixation initiale dépend donc du moyen de fixation. Celui-ci doit être capable de résister à la rupture en traction ainsi qu'au glissement de la greffe ligamentaire par rapport à la fixation. En outre, le système de fixation doit permettre de réduire au maximum la longueur du transplant de façon à éviter une distension élastique du transplant qui se produirait si sa longueur était très importante.

Un moyen de fixation couramment utilisé consiste à placer une vis d'interférence à chaque extrémité du tunnel. La vis introduite dans le tunnel en même temps que le transplant est de forme conique et vient comprimer le transplant à l'intérieur du tunnel. Malheureusement, les vis s'appuient sur l'os spongieux de moindre résistance. En outre, la vis d'interférence peut, par son filet agressif, abîmer le transplant lors de la mise en place.

Un autre moyen décrit dans le document EP 1146834B consiste à utiliser un crochet à l'extrémité inférieure du transplant retenu par la partie corticale de l'os. Mais ce simple crochet n'est pas suffisant dans la mesure où il est retenu par la partie la plus étroite de l'os à l'entrée du tunnel.

### Exposé de l'invention

C'est pourquoi le but de l'invention est d'utiliser comme moyen de fixation d'un transplant, un implant fixé au transplant et s'appuyant sur les parties pleines du pourtour extérieur de l'os se trouvant à l'entrée du tunnel.

L'objet de l'invention est donc un implant chirurgical utilisé pour fixer un transplant ligamentaire destiné à remplacer un ligament reliant deux os de part et d'autre d'une articulation. Le transplant ligamentaire est fixé dans un tunnel percé à partir de la paroi extérieure corticale du premier os et s'étendant sur une profondeur déterminée du deuxième os. L'implant comporte une première extrémité fixée au transplant et une seconde extrémité en forme de crochet s'appuyant sur le pourtour extérieur de la partie corticale du premier os se trouvant à l'entrée du tunnel. Le crochet comporte deux oreilles destinées à être situées de part et d'autre de l'entrée du tunnel de manière à ce que l'appui du crochet se fasse sur des parties pleines de l'os et non pas sur la partie d'épaisseur réduite à l'endroit du tunnel, l'axe des oreilles étant de préférence orthogonal au plan du crochet.

### Description brève des figures

Les buts, objets et caractéristiques de l'invention apparaîtront plus clairement à la lecture de la description qui suit faite en référence aux dessins dans lesquels :
la figure 1 représente une coupe de l'extrémité inférieure du fémur et de l'extrémité supérieure du tibia montrant le tunnel dans lequel a été fixé le transplant ligamentaire en utilisant un implant à appui extra cortical ;
la figure 2 représente une vue de face de l'extrémité inférieure du fémur et de l'extrémité supérieure du tibia en place montrant une coupe partielle du transplant illustré sur la figure 1 ;
la figure 3 représente une coupe de l'extrémité inférieure du fémur et de l'extrémité supérieure du tibia montrant le tunnel dans lequel a été fixé le transplant ligamentaire en utilisant deux broches non parallèles qui traversent le transplant ;
la figure 4 représente le dispositif ancillaire utilisé pour percer les tunnels destinés à recevoir les deux broches non parallèles ;
la figure 5 est une coupe sur deux plans du dispositif ancillaire montrant les deux mèches utilisées pour percer les tunnels destinés à recevoir les deux broches ; et
la figure 6 est une coupe sur deux plans de l'os et de la première branche du dispositif ancillaire montrant les implants en forme de broches placés dans les tunnels percés à l'aide des mèches.

### Description détaillée de l'invention

La description qui suit concerne une intervention chirurgicale ayant trait à une ligamentoplastie destinée à remplacer un des ligaments croisés antérieurs du genou reliant le fémur et le tibia. Mais il va de soi que cette intervention pourrait également être destinée à remplacer un ligament se trouvant dans une autre articulation que le genou.

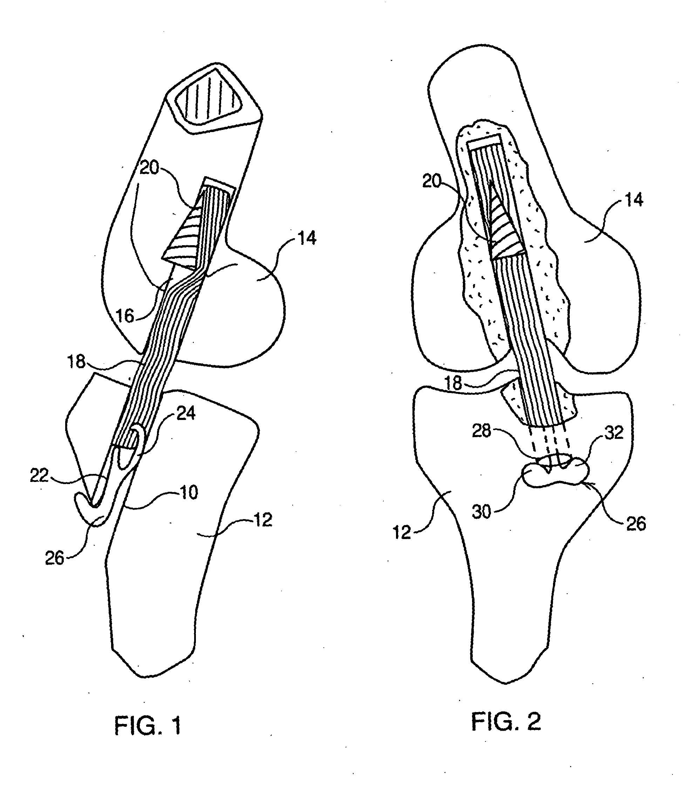

Comme illustré sur les figure 1 et 2, un tunnel 10 d'un diamètre compris entre 7mm et 12mm a été percé à partir de la paroi corticale du tibia 12 et traverse l'extrémité supérieure du tibia. Le tunnel 10 se prolonge dans le fémur 14 par la portion de tunnel 16 sur une longueur déterminée.

Un transplant 18 qui peut être un ligament artificiel ou une portion de ligament prélevée sur une autre partie du corps du patient ou sur un cadavre, est introduite dans le tunnel 10 et son prolongement 16. Une vis d'interférence 20 a été introduite après le transplant dans le prolongement de tunnel 16 de façon à venir bloquer l'extrémité supérieure du transplant. Comme on le voit sur la figure 1, la vis d'interférence 20 compresse le transplant ligamentaire contre la paroi du tunnel et déborde du tunnel du fait que la partie de l'os dans laquelle a été percé le tunnel est sa partie spongieuse. A noter que tout autre moyen de fixation pourrait être utilisé à la place de la vis d'interférence et en particulier les moyens de fixation décrits par la suite.

A l'extrémité inférieure du tunnel, le transplant ligamentaire 18 comporte un implant rigide monobloc 22 dont la partie supérieure 24 est en forme de boucle dans laquelle on a fait passer le transplant généralement replié sur lui-même avant de l'introduire dans le tunnel. La portion inférieure 26 de l'implant 22 est recourbée pour former une sorte de crochet. Lorsque le transplant 18 est introduit et enfoncé dans le tunnel, le crochet 26 vient en butée sur le pourtour extérieur 28 de l'entrée du tunnel 10. Ce pourtour extérieur étant constitué de la paroi corticale de l'os, il est rigide et retient l'implant 22 lorsqu'une force de traction est exercée par le transplant ligamentaire tiré vers le haut avant d'être fixé définitivement par la vis d'interférence 20.

Toutefois, un simple crochet sera retenu par la partie d'épaisseur réduite de l'os de l'entrée du tunnel sous-jacent qui risque de se briser sous l'effet de la force de traction exercée. Pour éviter cet inconvénient, le crochet 26 illustré sur la figure 2 comporte deux oreilles 30 et 32 situées due part et d'autre de l'entrée du tunnel 28 de manière à ce que l'appui du crochet se fasse sur des parties pleines de l'os, l'axe des oreilles étant de préférence orthogonal au plan du crochet.

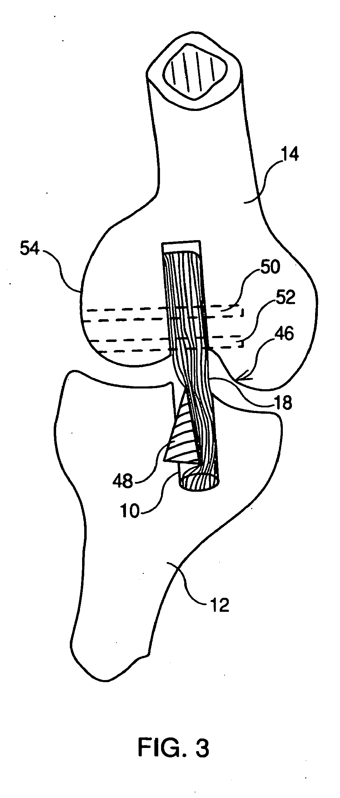

Une autre technique de fixation de la partie supérieure du transplant dans son tunnel est maintenant décrite en référence aux figures 3, 4, 5 et 6. Comme illustré sur la figure 3, le transplant est fixé dans sa partie supérieure à l'aide d'implants ayant la forme de broches 50 et 52 qui traversent le transplant 18 et qui sont introduites dans la partie corticale 54 du fémur 14 comme on va le voir ci-dessous. La particularité de ces broches est qu'elles ne sont pas parallèles mais forment un angle aigu entre elles et que la broche la plus basse prend appui sur la surface intérieure de la paroi corticale rigide 46 de la base du fémur 14. La partie inférieure du transplant peut être fixée par une vis d'interférence 48 ou tout autre moyen de fixation tel que l'implant illustré sur la figure 1.

A noter que cette technique de fixation par broches pourrait être utilisée pour la fixation de la partie inférieure du transplant, par exemple lorsque la partie supérieure du transplant est fixée par une tige transversale.

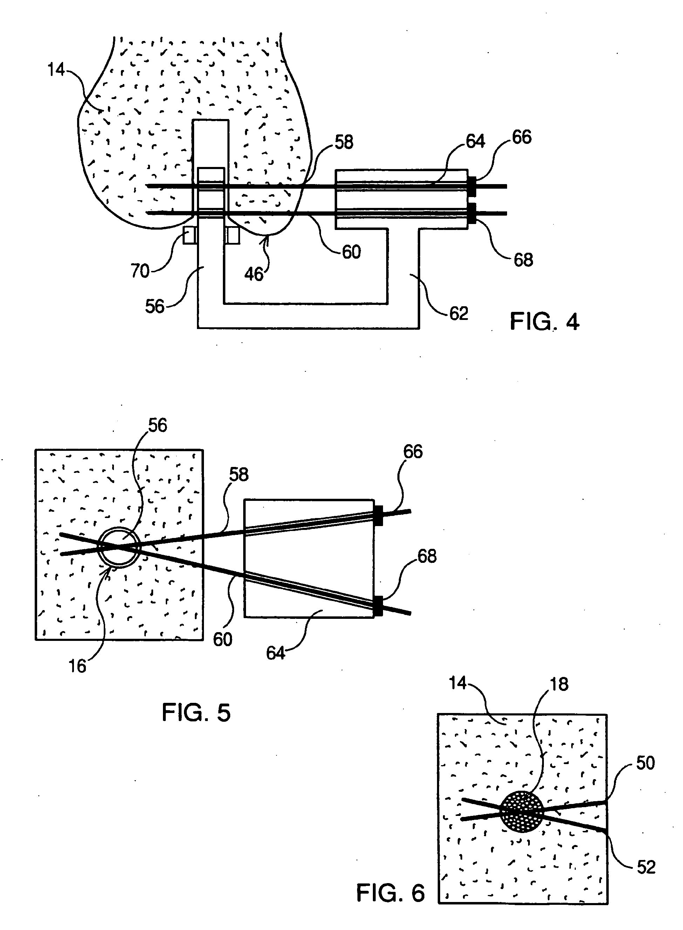

Le perçage des tunnels destinés à recevoir les broches 50 et 52 se fait à l'aide d'un dispositif ancillaire illustré sur la figure 4. Ce dispositif a une forme en U et comprend une première branche 56 introduite dans la portion de tunnel 16 située dans le fémur 14. Cette branche est percée de deux tunnels traversants situés à deux hauteurs différentes et destinés à être traversés par deux mèches de perceuse 58 et 60.

La deuxième branche du dispositif 62 est surmontée d'une platine 64 percée de deux tunnels traversants situés à des hauteurs différentes et formant un angle aigu entre eux comme le montre la figure 5. Les deux mèches 58 et 60 introduites dans les deux tunnels de la platine 64 servent à percer deux tunnels dans le fémur dans le but d'y introduire les deux implants en forme de broches 50 et 52. Lorsque les deux mèches 58 et 60 arrivent en fin de perçage, elles traversent la branche 56 et sont bloquées par deux butées 66 et 68 contre la platine 64.

La branche 56 est filetée et comporte un écrou 70 fileté également qui peut être descendu ou monté le long de la branche 56 de manière à ce que la mèche 60 perce un tunnel dans le fémur se trouvant juste au dessus de la surface interne de paroi corticale rigide 46 de l'os lorsque l'écrou est en butée contre la surface externe de la paroi corticale rigide 46.

Les deux mèches sont ensuite retirées et les implants en forme de broches 50 et 52 sont introduits dans les tunnels qui viennent d'être percés comme l'illustre la figure 6. Les deux broches forment donc un angle aigu entre elles et la broche 52 située juste au dessus de la paroi corticale est donc bloquée. En supposant que le tunnel soit plus haut que la paroi corticale, les broches qui se trouvent principalement dans la partie spongieuse de l'os vont descendre sous l'effet de la traction exercée par le transplant jusqu'à ce que la broche 52 atteigne la paroi corticale, mais avec difficulté dans la mesure où, les broches n'étant pas parallèles, elles sont situées dans des plans verticaux différents.

## Revendications

1. Implant chirurgical (22) utilisé pour fixer un transplant ligamentaire (18) reliant deux os de part et d'autre d'une articulation, ledit transplant ligamentaire étant fixé dans un tunnel formé par une première portion (10) percée à partir de la paroi extérieure corticale du premier os (12) et se prolongeant par une deuxième portion (16) sur une profondeur déterminée du deuxième os (14), ledit implant comportant une première extrémité (24) destinée à être fixée audit transplant et une seconde extrémité en forme de crochet (26) destinée à venir appuyer sur le pourtour extérieur (28) de la partie corticale dudit premier os se trouvant à l'entrée de ladite première portion de tunnel ;
ledit implant étant **caractérisé en ce que** ledit crochet comporte deux oreilles (30 et 32) destinées à être situées de part et d'autre de l'entrée dudit tunnel de manière à ce que l'appui dudit crochet se fasse sur des parties pleines de l'os et non pas sur la partie d'épaisseur réduite à l'endroit dudit tunnel, l'axe desdites oreilles étant de préférence orthogonal au plan dudit crochet.

2. Implant chirurgical (22) selon la revendication 1, dans lequel ladite première extrémité (24) est en forme de boucle dans laquelle on fait passer ledit transplant (18) replié sur lui-même avant de l'introduire dans ledit tunnel (10, 16).

3. Ensemble de fixation chirurgical d'un transplant ligamentaire (18) fixé à une extrémité par un implant selon l'une des revendications 1 ou 2, **caractérisé en ce qu'**il comprend en outre un moyen de fixation de l'autre extrémité dudit transplant constitué de deux broches (50, 52) destinées à être fixées transversalement audit transplant, les deux broches étant non parallèles et situées dans deux plans différents, la broche inférieure étant en appui sur la surface intérieure (46) de la partie corticale du deuxième os.

## Claims

1. Surgical implant (22) adapted to fix a ligament transplant (18) connecting two bones on either side of a joint, said ligament transplant being fixed in a tunnel formed by a first portion (10) pierced from the exterior cortical wall of a first bone (12) and extending through a second portion (16) at a pre-determined depth of a second bone (14), said implant including a first end (24) adapted to be fixed to said transplant and a second end in the form of a hook (26) destined to be supported against the outside rim (28) of the cortical part of said first bone found at the inlet of said first portion of the tunnel;
said implant being **characterized in that** said hook comprises two lugs (30 and 32) adapted to be located on either side of said tunnel inlet such that the hook rests on solid parts of the bone and not on the part of the bone having a reduced thickness at the position of the tunnel, the axe of said lugs being preferentially orthogonal to the plane of the hook.

2. The surgical implant (22) according to claim 1, wherein said first end (24) is in the form of a loop through which said transplant (18) is passed folded up on itself before introducing it into said tunnel.(10, 16).

3. Surgical fixation set for a ligament transplant (18) fixed at one end by an implant according to any one of claims 1 or 2, **characterized in that** it further comprises a means of fixation of the other end of said transplant constituted by two pins (50, 52) adapted to be fixed transversally on said transplant, the two pins being non-parallel and located on two different planes, the lower pin being supported against the interior surface (46) of the cortical part of the second bone.

## Patentansprüche

1. Chirurgisches Implantat (22), das zur Befestigung eines Bandtransplantats (18) verwendet wird, welches zwei Knochen zu beiden Seiten eines Gelenks verbindet, wobei das Bandtransplantat in einem Tunnel befestigt wird, der von einem ersten Abschnitt (10) gebildet wird, der ausgehend von der äußeren kortikalen Wand des ersten Knochens (12) gebohrt wird und sich durch einen zweiten Abschnitt (16) über eine bestimmte Tiefe des zweiten Knochens (14) verlängert, wobei das Implantat ein erstes Ende (24), das dazu bestimmt ist, am Transplantat befestigt zu werden, und ein zweites Ende in Form eines Hakens (26) aufweist, der dazu bestimmt ist, auf dem Außenumfang (28) des kortikalen Bereichs des ersten Knochens aufzuliegen, der sich am Eingang des ersten Tunnelabschnitts befindet;
wobei das Implantat **dadurch gekennzeichnet ist, dass** der Haken zwei Lappen (30 und 32) aufweist, die dazu bestimmt sind, sich zu beiden Seiten des Eingangs des Tunnels zu befinden, damit die Auflage des Hakens auf massiven Bereichen des Knochens und nicht in dem Bereich reduzierter Dicke an der Stelle des Tunnels erfolgt, wobei die Achse der Lappen vorzugsweise zur Ebene des Hakens orthogonal ist.

2. Chirurgisches Implantat (22) nach Anspruch 1, bei dem das erste Ende (24) die Form eines Rings hat, durch den das auf sich selbst umgebogene Transplantat (18) hindurch geführt wird, ehe es in den Tunnel (10, 16) eingeführt wird.

3. Chirurgische Befestigungseinheit für ein Bandtransplantat (18), das an einem Ende durch ein Implantat nach einem der Ansprüche 1 oder 2 befestigt wird, **dadurch gekennzeichnet, dass** sie außerdem eine Einrichtung zur Befestigung des anderen Endes des Transplantats enthält, die aus zwei Stiften (50, 52) besteht, welche dazu bestimmt sind, quer am Transplantat befestigt zu werden, wobei die zwei Stifte nicht parallel sind und sich in zwei unterschiedlichen Ebenen befinden, wobei der untere Stift auf der Innenfläche (46) des kortikalen Bereichs des zweiten Knochens aufliegt.
